# EUROPÄISCHE PATENTSCHRIFT

(11) **EP 0 838 469 B1**
(45) Veröffentlichungstag und Bekanntmachung des Hinweises auf die Patenterteilung: **09.01.2002**
(21) Anmeldenummer: 97118000.5
(22) Anmeldetag: 17.10.1997
(51) Int. Cl.: C07H 17/08, A61K 31/70

(54) **10,13,15-Trioxatricyclo(9.2.1.1. 9.6)-pentadecanon-Derivate, Verfahren zu ihrer Herstellung und diese Verbindungen enthaltende Arzneimittel**
10,13,15-Trioxatricyclo(9.2.1.1. 9.6)-pentadecanone derivatives, process for their preparation and medicament containing them
Dérivés du 10,13,15-Trioxatricyclo(9.2.1.1. 9.6)-pentadécanone, procédé pour leur préparation et médicaments les contenant

(30) Priorität: 24.10.1996 DE 19644195
(43) Veröffentlichungstag der Anmeldung: 29.04.1998
(73) Patentinhaber: Solvay Pharmaceuticals GmbH, 30173 Hannover (DE)
(72) Erfinder: Höltje, Dagmar, 30989 Gehrden (DE); Preuschoff, Ulf, 29275 Uelzen/Oldenstadt (DE); Finner, Emil, 30916 Isernhagen (DE); Eeckhout, Christian, 29690 Lindwedel (DE)
(74) Vertreter: Lauer, Dieter, Dr.

(56) Entgegenhaltungen:
- EP-A- 0 550 895

## Beschreibung

Die vorliegende Erfindung betrifft neue N-substituierte [(1'R),2R,3S,4S,5R,6R,9R,11R,12R,14R]-11-(1'-Hydroxypropyl)-3-[(2,6-dideoxy-3-C-methyl-3-O-methyl-α-L-ribo-hexopyranosyl)-oxy]-5-[(3,4,6-trideoxy-3-amino-β-D-xylohexopyranosyl)-oxy]-2,4,6,8,11,14-hexamethyl-10,13,15-trioxatricyclo[9.2.1.1^{9.6}]-pentadecan-1-on-Verbindungen mit motilin-agonistischen Eigenschaften und deren Säureadditionssalze sowie diese Verbindungen enthaltende pharmazeutische Zubereitungen und Verfahren zur Herstellung dieser Verbindungen. Die erfindungsgemäßen Verbindungen sind ringverengte N-Desmethyl-N-isopropylspiroacetalderivate des Erythromycin A.

Das Antibiotikum Erythromycin A besitzt bekanntermaßen neben seinen antibiotischen Wirkungen auch für Antibiotika unerwünschte gastrointestinale Nebenwirkungen, u. a. eine starke Vermehrung der Kontraktionsaktivität im Magen-Darm-Bereich mit Magen- und Darmkrämpfen, Übelkeit, Erbrechen und Diarrhöe.

Es hat mehrere Versuche gegeben, das Erythromycin A so abzuwandeln, daß Derivate erhalten werden, in denen die antibiotische Wirkung praktisch nicht mehr vorhanden ist, jedoch eine die Motilität des gastrointestinalen Traktes beeinflussende Wirkung erhalten ist. Aus der EP-Patentanmeldung 0 550 895 sind ringverengte N-Desmethyl-N-isopropyl-erythromycin-A-Derivate mit gastrointestinal wirksamen motilin-agonistischen Eigenschaften bekannt. Der vorliegenden Erfindung liegt die Aufgabe zugrunde, neue oral wirksame ringverengte Derivate des Erythromycin A ohne Antibiotikawirkung und mit die Motilität des gastrointestinalen Traktes günstig beeinflussenden Eigenschaften mit einem verbesserten Wirkprofil zu entwickeln.

Es wurde nun gefunden, daß die neuen ringverengten N-Desmethyl-N-isopropyl-spiroacetal-Derivate des Erythromycin A selektive motilin-agonistische Eigenschaften aufweisen und die Motilität des gastrointestinalen Traktes in günstiger Weise stimulieren und den Tonus des unteren Oesophagus Sphincter und den Tonus des Magens verstärkende Wirkungen zeigen. Aufgrund ihres Wirkungsprofils eignen sich die erfindungsgemäßen Substanzen zur Behandlung von Motilitätsstörungen im gastrointestinalen Trakt und zeichnen sich dabei durch eine gute Verträglichkeit, gute orale Wirksamkeit und gute Stabilität aus.

Die vorliegende Erfindung betrifft daher neue [(1'R),2R,3S,4S,5R,6R,9R,11R,12R,14R]-11-(1'Hydroxypropyl)-2,4,6,8,11,14-hexamethyl-10,13,15-trioxatricyclo[9.2.1.1^{9.6}]-pentadecan-1-on-Derivate der allgemeinen Formel I 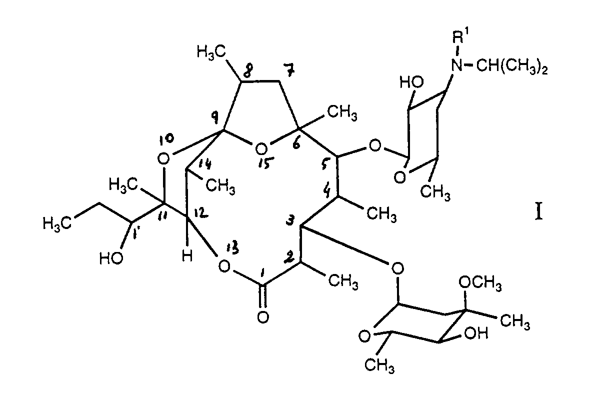 worin R¹ Methyl oder Wasserstoff bedeutet, und deren stabile und physiologisch verträgliche Säureadditionssalze.

Als günstig erweist sich insbesondere die Verbindung der Formel I, worin R¹ Methyl bedeutet.

Die Verbindungen der Formel I können erhalten werden, indem man auf an sich bekannte Weise [2R(2'R,3'R),3S,4S,5R,6R,10R,11R]-11-(2',3'-Dihydroxypent-2'-yl)-2,4,6,8,10-pentamethyl-12,13-dioxabicyclo[8.2.1] tridec-8-en-1-on-Derivate-Verbindungen der allgemeinen Formel II 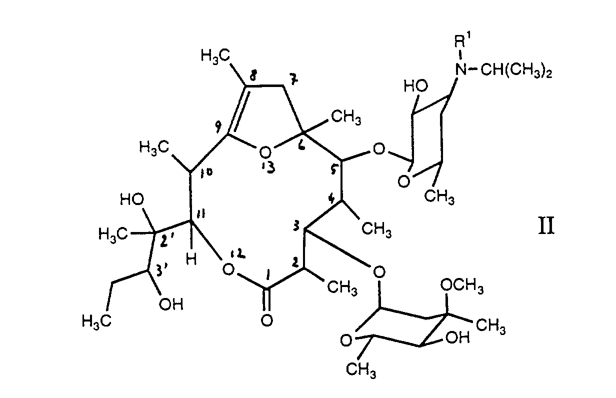 worin R¹ obige Bedeutung besitzt, durch Säurebehandlung in Verbindungen der Formel I überführt und gewünschtenfalls in die erhaltene Verbindung der Formel I, worin R¹ Wasserstoff bedeutet, einen Methylrest R¹ einführt oder in der erhaltenen Verbindung der Formel I, worin R¹ Methyl bedeutet, den Methylrest R¹ abspaltet, und gewünschtenfalls freie Verbindungen der Formel I in ihre stabilen Säureadditionssalze überführt oder die Säureadditionssalze in die freien Verbindungen der Formel I überführt.

Die Verbindungen der Formel I werden durch protonenkatalysierte intramolekulare Spirocyclisierung aus Verbindungen der Formel II erhalten. Die Spirocyclisierung erfolgt auf an sich bekannte Weise durch Behandeln mit Säuren, vorzugsweise in wäßrigem Medium, bei niederen pH-Werten, beispielsweise pH-Werten von höchstens pH 3, zweckmäßigerweise bei pH-Werten zwischen 1,5 und 3. Als Säuren können gegenüber den übrigen funktionellen Gruppen der Verbindungen der Formel I und II inerte wasserlösliche anorganische oder organische Säuren eingesetzt werden. Es ist zweckmäßig, ein Absinken des pH-Wertes unter 1 zu vermeiden, damit keine Hydrolysenebenreaktionen auftreten. Geeignete Reaktionsmedien sind beispielsweise wäßrige Salzsäurelösung oder wäßrige Essigsäurelösung. Vorteilhaft findet die Cyclisierungsreaktion in wäßriger Salzsäurelösung bei Raumtemperatur statt.

Die erhaltene Verbindung der Formel I, worin R¹ Wasserstoff bedeutet, kann gewünschtenfalls nachträglich in an sich bekannter Weise zu der entsprechenden N-Methyl-Verbindung alkyliert werden. Die Alkylierung kann auf an sich bekannte Weise durch Umsetzen mit einem Methylhalogenid oder als reduktive Alkylierung durch Umsetzen mit Formaldehyd unter reduzierenden Bedingungen erfolgen und kann beispielsweise bei den nachstehend zur Alkylierung der Verbindungen der Formel III angegebenen Bedingungen durchgeführt werden.

Aus der Verbindung der Formel I, worin R¹ Methyl bedeutet, kann der Methylrest R¹ gewünschtenfalls nachträglich abgespalten werden. Die Demethylierung kann auf an sich bekannte Weise durch Behandeln der Verbindung mit einem Halogen, insbesondere Jod und/oder Brom, in einem inerten Lösungsmittel in Gegenwart einer geeigneten Base erfolgen. Als Basen eignen sich beispielsweise Alkalimetallalkoholate, Alkalimetallhydroxide und Alkalimetallsalze von schwachen organischen Säuren.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in pharmakologisch verträgliche Säureadditionssalze überführt werden. Zur Vermeidung von Hydrolysenebenreaktionen ist es zweckmäßig, zur Salzbildung nur äquivalente Mengen Säuren zu verwenden.

Als pharmakologisch annehmbare Säureadditionssalze der Verbindungen der Formel I eignen sich beispielsweise deren Salze mit anorganischen Säuren, z. B. Kohlensäure, Halogenwasserstoffsäuren, insbesondere Chlorwasserstoffsäure, oder mit organischen Säuren, beispielsweise niederen aliphatischen Mono- oder Dicarbonsäuren wie Maleinsäure, Fumarsäure, Milchsäure, Weinsäure oder Essigsäure.

An dem durch die Spirocyclisierungsreaktion entstandenen Chiralitätszentrum, dem Kohlenstoffatom in Position 8, können zwei epimere Formen auftreten, so daß zwei Isomere der Verbindungen der Formel I möglich sind. Die vorliegende Erfindung umfaßt sowohl das Isomerengemisch als auch die reinen isomeren Verbindungen der Formel I. Bei der Ringschlußreaktion entsteht ein Isomerengemisch. Die reinen Isomeren können aus diesem Gemisch in an sich bekannter Weise durch übliche Trennverfahren, beispielsweise durch chromatographische Trennung erhalten werden.

Die Ausgangsverbindungen der Formel II sind aus der EP 0 550 895 A bekannt und können nach den dort beschriebenen Verfahren hergestellt werden. So können Verbindungen der Formel II erhalten werden, indem in Verbindungen der allgemeinen Formel III 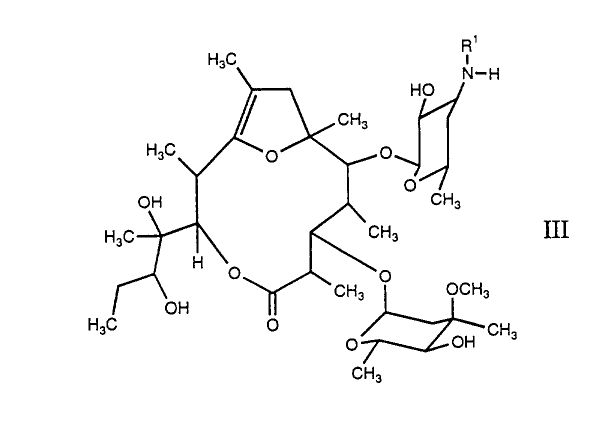 worin R¹ obige Bedeutung besitzt, auf an sich bekannte Weise ein Isopropylrest eingeführt wird.

Zur Einführung des Isopropylrestes können die Verbindungen der Formel III auf an sich bekannte Weise alkyliert werden. Vorzugsweise wird die Alkylierung als reduktive Alkylierung auf an sich bekannte Weise durch Umsetzen der Verbindungen der Formel III mit Aceton unter reduzierenden Bedingungen durchgeführt. Beispielsweise können die Verbindungen der Formel III mit Aceton in Gegenwart eines Reduktionsmittels, beispielsweise einer komplexen Borhydridverbindung wie Natriumcyanoborhydrid, Natriumtriacetoxyborhydrid oder Natriumborhydrid, umgesetzt werden. Gewünschtenfalls kann die Alkylierung, insbesondere derjenigen Verbindung der Formel III, worin R¹ Methyl bedeutet, auch durch Umsetzen mit einem Isopropylhalogenid, insbesondere Isopropyljodid, oder Isopropylsulfat oder einem Isopropylsulfonsäureester erfolgen. Zweckmäßigerweise wird die Alkylierung in einem unter den Reaktionsbedingungen inerten organischen Lösungsmittel durchgeführt. Für die reduktive Alkylierung kann beispielsweise ein Überschuß an Aceton als Lösungsmittel dienen. Ferner eignen sich als Lösungsmittel auch cyclische Ether, wie Tetrahydrofuran oder Dioxan, aromatische Kohlenwasserstoffe wie Toluol oder auch niedere Alkohole. Die Alkylierung kann bei Temperaturen zwischen Raumtemperatur und Siedetemperatur des Lösungsmittels erfolgen. Bei der Alkylierung mit einem Isopropylderivat, beispielsweise einem Isopropylhalogenid wie Isopropyljodid, wird zweckmäßigerweise in Gegenwart einer Base wie beispielsweise einem Alkalimetallcarbonat oder einem tert. organischen Amin gearbeitet.

Gewünschtenfalls kann in eine erhaltene Verbindung der Formel II, worin R¹ Wasserstoff bedeutet, ein Methylrest R¹ eingeführt oder in einer erhaltenen Verbindung der Formel II, worin R¹ Methyl bedeutet, der Methylrest R¹ abgespalten werden. Derartige Methylierungen oder Demethylierungen können auf an sich bekannte Weise, beispielsweise unter den für die Einführung oder Abspaltung einer Methylgruppe in den Verbindungen der Formel I beschriebenen Bedingungen erfolgen.

Die Verbindungen der Formel III können ausgehend von Erythromycin A der Formel IV 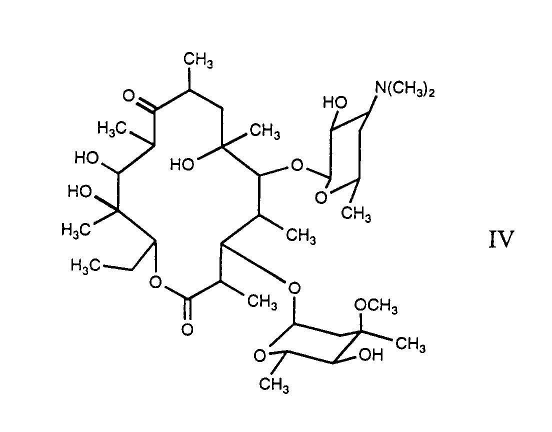 nach an sich bekannten Methoden erhalten werden. So kann das Erythromycin A zunächst auf an sich bekannte Weise, beispielsweise nach dem aus der DE-OS 21 54 032 bekannten Verfahren, mono- oder didemethyliert werden durch Umsetzung mit Halogen, bevorzugt Jod, in einem inerten Lösungsmittel in Gegenwart einer geeigneten Base. Als Basen eignen sich beispielsweise Alkalimetallalkoholate, Alkalimetallhydroxide, Alkalimetallcarbonate und Alkalimetallsalze von schwachen Carbonsäuren wie beispielsweise Alkalimetallacetate oder -propionate. Es können 1 bis 10 Äquivalente des Halogens bezogen auf die Menge an zu demethylierender Erythromycin-Verbindung eingesetzt werden. Für die Monodemethylierung werden als Basen vorzugsweise Alkalimetallhydroxide und/oder -salze verwendet. Die Menge der Base wird vorzugsweise so gewählt. daß ein pH-Wert im Bereich von 5 bis 9 gewährleistet ist. Als Lösungsmittel eignen sich Methanol, cyclische Ether wie Dioxan oder Tetrahydrofuran, Dimethylformamid oder Mischungen der genannten Lösungsmittel mit Wasser. Die Monodemethylierung wird zweckmäßigerweise bei Temperaturen zwischen Raumtemperatur und 50 °C durchgeführt. Die Reaktion kann durch Bestrahlung mit Licht, z. B. Licht mit einer Wellenlänge von über 290 nm aus einer Quecksilberniederdrucklampe mit einem Filter aus Quarz oder hitzebeständigem Glas (z. B. Pyrex ^{R}) gefördert werden. Die Didemethylierung wird vorzugsweise in einem trockenen niederen Alkohol, z. B. Methanol, in Gegenwart des entsprechenden Alkalimetallalkoholates bei Temperaturen zwischen 0 und 10 °C durchgeführt. Gewünschtenfalls kann zur Herstellung des didemethylierten Produktes auch von bereits monodemethyliertem Produkt ausgegangen werden.

Das mono- oder didemethylierte Erythromycin A kann auf an sich bekannte Weise durch milde Säurebehandlung in ein entsprechendes mono- oder didemethyliertes 8,9-Anhydroerythromycin-A-6,9-hemiketal der allgemeinen Formel V 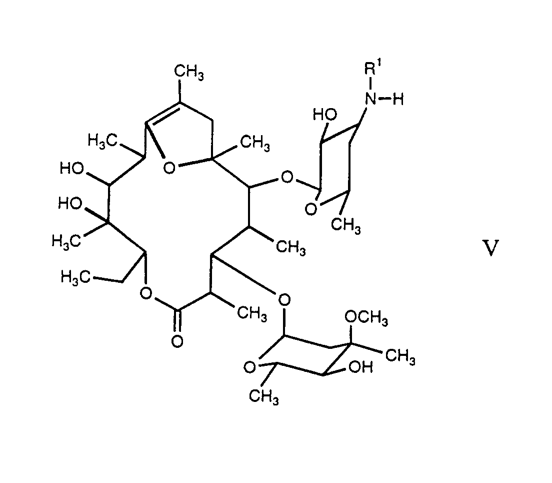 worin R¹ Wasserstoff oder Methyl bedeutet, überführt werden. Die Hemiketalbildung kann beispielsweise durch Behandeln mit einer organischen Säure wie Zitronensäure, Ameisensäure oder Eisessig oder verdünnter Mineralsäure bei Temperaturen zwischen Raumtemperatur und ca. 50 °C erfolgen.

In den Verbindungen der Formel V kann auf an sich bekannte Weise durch intramolekulare Translactonisierung eine Ringverengung des 14-gliedrigen Lactonringes des Erythromycin-Gerüstes zu einem 12-gliedrigen Lactonring unter Bildung der entsprechenden Verbindungen der Formel III durchgeführt werden. Hierzu werden die Verbindungen der Formel V auf an sich bekannte Weise in einem niederen Alkohol in Gegenwart einer Base erhitzt, beispielsweise auf Temperaturen zwischen 40 °C und 70 °C, vorzugsweise Siedetemperatur des Reaktionsgemisches. Als Basen eignen sich insbesondere Alkalimetallcarbonate, aber auch organische Basen wie tertiäre Amine, insbesondere tertiäre Niederalkylamine. Bei dieser Ringverengung ändert sich die Konfiguration der Chiralitätszentren nicht.

Die neuen Verbindungen der Formel I und ihre physiologisch verträglichen Säureadditionssalze besitzen interessante pharmakologische Eigenschaften, insbesondere die Motilität des gastrointestinalen Traktes stimulierende motilin-agonistische Eigenschaften. Dabei zeichnen sie sich durch ein günstiges Wirkprofil mit guter oraler Wirksamkeit aus. Sie sind frei von antibiotischen Wirkungen und besitzen eine hohe selektive Affinität zu Motilin-Rezeptoren, während sie in motilin-agonistisch wirksamen Dosisbereichen keine praktisch relevante Affinität zu anderen Rezeptoren im gastrointestinalen Trakt wie Adrenalin-, Acetylcholin-, Histamin-, Dopamin- oder Serotonin-Rezeptoren zeigen. Die Verbindungen weisen eine überraschend gute Leberverträglichkeit auf, welche sie für Anwendungen über längere Zeiträume hin geeignet macht.

Um eine geregelte Verdauung der eingenommenen Nahrung zu gewährleisten, wirken im gesunden Zustand das autonome Nervensystem und Hormone des gastrointestinalen Traktes zusammen, um eine geregelte Kontraktionstätigkeit des gastrointestinalen Traktes nicht nur direkt nach Nahrungsaufnahme, sondern auch bei leerem gastrointestinalen Trakt zu erzeugen. Motilin ist ein bekanntes gastrointestinales Peptidhormon, welches die Motilität des gastrointestinalen Traktes stimuliert und eine koordinierte Motilität im gesamten gastrointestinalen Trakt im nüchternen Zustand sowie nach Nahrungsaufnahme induziert.

Die Verbindungen der Formel I zeigen motilin-artige physiologische Wirkungen, indem sie als Agonisten für Motilinrezeptoren wirksam werden. So zeigen die Verbindungen der Formel I ausgeprägte stimulierende Wirkungen im Magen-Darm-Bereich und am unteren Speiseröhrensphincter. Sie bewirken insbesondere eine Beschleunigung der Magenentleerung, eine Erhöhung des Magentonus und eine langanhaltende Erhöhung des Ruhe-Tonus des Oesophagus Sphincter. Aufgrund ihres motilinartigen Wirkungsprofils eignen sich die Substanzen zur Behandlung von Krankheitszuständen, welche mit Motilitätsstörungen im gastrointestinalen Trakt und/oder Rückfluß von Speisebrei aus dem Magen in die Speiseröhre verbunden sind. So sind die Verbindungen der Formel I z. B. indiziert bei Gastroparesis verschiedensten Ursprungs, Störungen des Magentonus, Störungen der Magenentleerung und gastroösophagalem Rückfluß, Dyspepsie und postoperativen Motilitätsstörungen.

Die gastrointestinal wirksamen Eigenschaften der Verbindungen der Formel I lassen sich in pharmakologischen Standardtestmethoden in vitro und in vivo nachweisen.

### Beschreibung der Testmethoden.

### 1. Bestimmung des Bindungsvermögens der Testsubstanzen an Motilin-Rezeptoren.

Die Affinität der Verbindungen der Formel I an Motilin-Rezeptoren wird in vitro an einer Fraktion eines Gewebehomogenats aus dem Kaninchenantrum gemessen. Bestimmt wird die Verdrängung von radioaktiv markiertem jodiertem Motilin aus der Motilin-Rezeptor-Bindung durch die Testsubstanzen.

Die Rezeptor-Bindungsstudien werden nach einer Modifikation der Methode von Borman et al. (Regulatory Peptides 15 (1986), 143-153) durchgeführt. Zur Herstellung des ¹²⁵Jodmarkierten Motilins wird Motilin auf an sich bekannte Weise, z. B. analog der von Bloom et al. (Scand. J. Gastroenterol. 11 (1976) 47 - 52) beschriebenen Methode enzymatisch unter Verwendung von Lactoperoxidase jodiert.

Zur Gewinnung der in dem Test verwendeten Gewebehomogenatfraktion aus dem Kaninchenantrum wird das von Schleimhäuten befreite Antrum zerkleinert und im 10-fachen Volumen einer kalten Homogenisierungspufferlösung (50 mM Tris-HCl-Puffer, 250 mM Sucrose, 25 mM KCl, 10 mM MgCl₂, pH 7,4) mit Zusatz von Inhibitoren (1 mM Jodacetamid, 1 µM Pepstatin, 0,1 mM Methylsulfonylfluorid, 0,1 g/l Trypsininhibitor, 0,25 g/l Bacitracin) mit einem Homogenisator 15 sec. bei 1500 Umdrehungen pro Minute homogenisiert. Das Homogenisat wird dann 15 Minuten lang bei 1000 g zentrifugiert, der erhaltene Rückstand wird viermal mit Homogenisierungspufferlösung gewaschen und schließlich in 0,9%-iger Natriumchloridlösung (in einem der 5-fachen Gewichtsmenge des Antrums entsprechendem Volumen) resuspendiert. Die so erhaltene Gewebefraktion, welche als "rohe Membranzubereitung" bezeichnet wird, wird für den Test eingesetzt.

Für den Bindungsversuch werden 200 µl der rohen Membranfraktion (0,5 - 1 mg Protein) in 400 µl einer Pufferlösung A (50 mM Tris-HCl-Puffer, 1,5 % BSA, 10 mM MgCl₂, pH 8,0) mit 100 µl jodiertem Motilin in Pufferlösung B (10 mM Tris-HCl-Puffer, 1 % BSA, pH 8) verdünnt (Endkonzentration 50 pM) 60 min. bei 30 °C inkubiert. Die Reaktion wird durch Zugabe von 3,2 ml kalter Pufferlösung B gestoppt und gebundenes und nichtgebundenes Motilin werden durch Zentrifugieren (1000 g, 15 Minuten) voneinander getrennt. Der nach dem Zentrifugieren als Pellet erhaltene Rückstand wird mit Pufferlösung B gewaschen und in einem Gamma-Zähler ausgezählt. Die Verdrängungsstudien werden durch Zugabe steigender Mengen der zu testenden Substanz in das Inkubationsmedium durchgeführt. Als Test-substanzlösungen werden wäßrige Lösungen eingesetzt, welche durch geeignete Verdünnung von 60 x 10⁻⁴-molaren wäßrigen Stammlösungen hergestellt werden. In Wasser schwer lösliche Testsubstanzen werden zunächst in 60%-igem Äthanol gelöst und diese Lösung wird mit soviel Wasser verdünnt, daß in der zu testenden Lösung die Äthanolkonzentration 1,6 Vol.-% nicht übersteigt. Aus den erhaltenen Meßdaten wird als IC₅₀ der jeweiligen Testsubstanz diejenige Konzentration bestimmt, welche eine 50%-ige Hemmung der spezifischen Bindung des jodierten Motilin an die Motilin-Rezeptoren bewirkt. Aus dieser wird der entsprechende pIC₅₀-Wert berechnet. Nach der vorstehenden Methode wurde für die Substanz des Beispiels 1 ein pIC₅₀-Wert von 7,85 bestimmt.

### 2. In-vivo-Bestimmung des Einflusses der Substanzen auf den Magentonus

Der Magentonus spielt eine wichtige Rolle bei der Magenentleerung. Ein erhöhter Magentonus trägt zu einer beschleunigten Magenentleerung bei.

Der Einfluß von Substanzen auf den Magentonus wird an Beagle-Hunden mit Hilfe eines Barostaten bestimmt, welcher mit einem Kunststoffbeutel im Magen des Hundes verbunden ist und die Messung von Volumen oder Druck im Magen des Hundes ermöglicht. Mit dem Barostaten wird das Magenvolumen bei konstantem Druck im Magen oder der Magendruck bei konstantem Volumen im Magen bestimmt. Bei Erhöhung des Magentonus stellt man bei einem bestimmten Druck ein verringertes Magenvolumen fest und einen erhöhten Druck bei einem bestimmten Volumen. In dem zur Untersuchung der durch die Substanzen bewirkten Erhöhung des Magentonus verwendeten Testmodell wird die durch die Substanzen verursachte Magenvolumenänderung bei konstantem Druck gemessen. Der Magen der Versuchstiere wird durch Lipideinnahme relaxiert, d.h. der Magentonus sinkt wodurch das Magenvolumen entsprechend zunimmt. Als Maß für die Magentonus erhöhende Wirkung der Substanzen wird die nach Substanzeinnahme durch Wiederanstieg des Magentonus eintretende Reduktion des durch Lipidgabe vergrößerten Magenvolumens in % gemessen. Die Substanz des Beispiels 1 zeigte in diesem Testmodell in der maximal tolerierbaren Dosis eine Reduktion des nach Lipidgabe vergrößerten Magenvolumens um 69 %.

Aufgrund ihrer Wirkungen im gastrointestinalen Trakt sind die Verbindungen der Formel I in der Gastroenterologie als Arzneimittel für größere Säugetiere, insbesondere Menschen, zur Prophylaxe und Behandlung von Motilitätsstörungen des gastrointestinalen Traktes geeignet.

Die zu verwendenden Dosen können individuell verschieden sein und variieren naturgemäß je nach Art des zu behandelnden Zustandes und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 1 bis 100 mg pro Einzeldosis.

Als Heilmittel können die Verbindungen der Formel I mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe, wie z. B. Milchzukker, Stärke oder Talkum oder flüssiger Verdünnungsmittel, wie z. B. Wasser, fetten Ölen oder flüssigen Paraffinen und unter Verwendung von pharmazeutisch üblichen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

### Beispiel 1:

[(1'R),2R,3S,4S,5R,6R,9R,11R,12R,14R]-11-(1'-Hydroxypropyl)-3-[(2,6-dideoxy-3-C-methyl-3-O-methyl-α-L-ribo-hexopyranosyl)-oxy]-5-[(3,4,6-trideoxy-3-(N-methyl-N-isopropylamino)-β-D-xylohexopyranosyl)-oxy]-2,4,6,8,11,14-hexamethyl-10,13,15-tri-oxatricyclo[9.2.1.1^{9.6}]-pentadecan-1-on (= Isomerengemisch der Verbindung der Formel I, R¹ = Methyl).

### A) Herstellung von N-Desmethylerythromycin A.

20 g Erythromycin A (= 27,2 mmol) und 11,2 g (= 136,2 mmol) Natriumacetat wurden in 200 ml eines Gemisches aus Methanol/ Wasser 8:2 gelöst. Die Lösung wurde auf 47 °C erwärmt. Dann wurden 6,9 g (= 136,2 mmol) Jod zugegeben. Der pH-Wert wurde durch Zugeben von verdünnter wäßriger Natriumhydroxidlösung auf 8 bis 9 gehalten. Nach 3 Stunden wurde das Reaktionsgemisch zur Aufarbeitung in ein Gemisch aus 1 l Wasser und 20 ml Ammoniumhydroxidlösung gegossen. Das Reaktionsgemisch wurde mit Essigsäureethylester extrahiert, und der organische Extrakt mit Ammoniumhydroxid-haltigem Wasser gewaschen und eingeengt. Das nach Entfernen des Lösungsmittels verbleibende Rohprodukt wurde aus Aceton/Ammoniumhydroxidlösung 50:3 umkristallisiert. Schmelzpunkt 143-148 °C.

### B) Herstellung von N-Desmethyl-8,9-anhydroerythromycin-A-6,9-hemiketal (= Verbindung der Formel V, R¹ = Methyl).

21 g des unter A) erhaltenen Produktes wurden in 110 ml Eisessig gelöst und die Lösung wurde 1 Stunde bei Raumtemperatur gerührt. Dann wurde das Reaktionsgemisch zur Aufarbeitung unter Eiskühlung in 400 ml konzentrierte Ammoniumhydroxidlösung getropft. Das Reaktionsgemisch wurde mit Essigsäureethylester extrahiert, der organische Extrakt mit Wasser gewaschen und das Lösungsmittel abgezogen. Das als Rückstand verbleibende Rohprodukt wurde erst aus Ether und dann aus Methanol umkristallisiert. Es wurden 14 g reines Produkt mit einem Schmelzpunkt von 145 °C erhalten.

### C) Herstellung von [2R(2'R,3'R),3S,4S,5R,6R,10R,11R]-11-(2',3'-Dihydroxypent-2'-yl)-3-[(2,6-dideoxy-3-C-methyl-3-O-methyl-α-L-ribo-hexpoyranosyl)-oxy]-5-[(3,4,6-trideoxy-3-methylamino-β-D-xylo-hexopyranosyl)-oxy]-2,4,6,8,10-pentamethyl-12,13-dioxabicyclo[8.2.1]tridec-8-en-1-on (= Verbindung der Formel III, R¹ = Methyl).

9,4 g (= 13,4 mmol) des unter B) erhaltenen Produktes wurden mit 1,9 g (= 13,4 mmol) Kaliumcarbonat in Methanol 2,5 Stunden unter Rückfluß gekocht. Zur Aufarbeitung wurde das Reaktionsgemisch eingeengt, mit Wasser verdünnt und mit Essigsäureethylester extrahiert. Das nach Entfernen des Lösungsmittels verbleibende Rohprodukt wurde aus Isopropanol umkristallisiert. Es wurden 7,1 g reines Produkt mit einem Schmelzpunkt von 199 bis 200 °C erhalten, optischer Drehwert
[α]²⁰_{D}:-31,6 ° (c = 1, Methanol).

### D) Herstellung von [2R(2'R,3'R),3S,4S,5R,6R,10R,11R]-11-(2',3'-Dihydroxypent-2'-yl)-3-[(2,6-dideoxy-3-C-methyl-3-O-methyl-α-L-ribohexopyranosyl)-oxy]-5-[(3,4,6-trideoxy-3-(N-methyl-N-isopropylamino)-β-D-xylohexopyranosyl)-oxy]-2,4,6,8,10-pentamethyl-12,13-dioxabicyclo[8.2.1]tridec-8-en-1-on (= Verbindung der Formel II, R¹ = Methyl).

2 g (= 2,8 mmol) des vorstehend unter C) erhaltenen Produktes wurden in Methanol gelöst und der pH-Wert der Lösung wurde durch Zugabe von verdünnter Salzsäurelösung auf 4 eingestellt. Zu der Lösung wurden 2 g eines Molekularsiebs (Calciumaluminiumsilikat, Porendurchmesser 4 Å) ein Überschuß an Aceton und 0,4 g (= 6,4 mmol) Natriumcyanoborhydrid gegeben. Das Reaktionsgemisch wurde 12 Stunden gerührt. Zur Aufarbeitung wurde von dem Molekularsieb abfiltriert, das Filtrat eingeengt, mit Wasser versetzt und mit Essigsäureethylester extrahiert. Das nach Einengen des Essigsäureethylesterextraktes als Rückstand verbleibende Rohprodukt wurde säulenchromatographisch über Kieselgel (Elutionsmittel Essigsäureethylester/Methanol 95:5) gereinigt. Es wurden 1,4 g des gereinigten Produktes mit einem Schmelzpunkt von 130 bis 134 °C erhalten, optischer Drehwert [α]²⁰_{D}:-32,8°.

### E) Herstellung der Titelverbindung

30 g des vorstehend unter D) erhaltenen Produktes wurden in 2250 ml Wasser gegeben. Unter Rühren wurde zu dem Gemisch konzentrierte Salzsäure bis zum Erreichen eines pH-Wertes von 2-3 zugetropft. Anschließend wurde das Reaktionsgemisch 7 Stunden bei Raumtemperatur gerührt. Zur Aufarbeitung wurde dem Reaktionsgemisch konzentrierte Ammoniaklösung bis zum Erreichen von pH 11 zugesetzt. Anschließend wurde das Reaktionsgemisch mit Dichlormethan extrahiert. Der organische Extrakt wurde eingeengt. Das nach Einengen des Dichlormethanextraktes verbleibende Rohprodukt wurde durch Umkristallisation aus Acetonitril gereinigt. Es wurden 19,6 g der Titelverbindung mit einem Schmelzpunkt von 181 bis 183 °C erhalten, optischer Drehwert [α]²⁰_{D}:-52,2°.

### Isomerenauftrennung:

Die Auftrennung der Isomeren erfolgte durch semipräparative Hochleistungs-Flüssigkeitschromatographie (= High Performance liquid Chromatography, abgekürzt als HPLC) auf einer Fertigsäule mit den Abmessungen 300 mm (L) x 7,8 mm (ID) der Firma Waters. Es wurde das Reversed-Phase-Säulenmaterial "Symmetry-Prep® " C18 (7µm) verwendet. Als Eluens diente ein Gemisch aus 600 ml einer wäßrigen 0,05 M KH₂PO₄-Lösung mit einem pH-Wert von 6.0 (eingestellt mit einer 1M NaOH-Lösung) und 400 ml Acetonitril.

Mit einer Retentionszeit von 5,2 Minuten wurde das 8R-Isomer erhalten.

Mit einer Retentionszeit von 6,8 Minuten wurde das 8S-Isomer erhalten.

### Beispiel 2:

[(1'R),2R,3S,4S,5R,6R,9R,11R,12R,14R]-11-(1'-Hydroxypropyl)-3-[(2,6-dideoxy-3-C-methyl-3-O-methyl-α-L-ribohexopyranosyl)-oxy]-5-[(3,4,6-trideoxy-3-(N-isopropylamino)-β-D-xylohexopyranosyl)-oxy]-2,4,6,8,11,14-hexamethyl-10,13,15-trioxatricyclo[9.2.1.1^{9.6}]-pentadecan-1-on (= Isomerengemisch der Verbindung der Formel I, R¹ = Wasserstoff).

### A) Herstellung von [2R(2'R,3'R),3S,4S,5R,6R,10R,11R]-11-(2',3'-Dihydroxypent-2'-yl)-3-[(2,6-dideoxy-3-C-methyl-3-O-methyl-α-L-ribohexopyranosyl)-oxy]-5-[(3,4,6-trideoxy-3-(N-isopropylamino)-ß-D-xylohexopyranosyl)-oxy]-2,4,6,8,10-pentamethyl-12,13-dioxabicyclo[8.2.1]tridec-8-en-1-on.

Eine Mischung aus 7,3 g Natriummethylat und 500 ml Methanol wurde unter Stickstoffatmosphäre auf 0 °C gekühlt. Dann wurde eine Lösung von 20 g der in Beispiel 1D) erhaltenen Verbindung der Formel II (R¹ = Methyl) in 100 ml Methanol dazugetropft. Anschließend wurden 34,1 g Jod portionsweise zugegeben und das Reaktionsgemisch wurde 24 Stunden bei einer Temperatur von 0 bis 5 °C gehalten. Zur Aufarbeitung wurde das Reaktionsgemisch in eine Lösung von 58 g Natriumthiosulfat und 48 ml konzentrierter Ammoniaklösung in 1,5 l Wasser gegeben. Die wäßrige Phase wurde 4-mal mit je 100 ml Chloroform extrahiert. Die vereinigten organischen Phasen wurden einmal mit einem Gemisch aus 5 ml konzentrierter Ammoniaklösung und 100 ml Wasser gewaschen, über Natriumsulfat getrocknet und eingeengt. Der verbleibende Rückstand wurde säulenchromatographisch an Kieselgel gereinigt. Es wurden 0,5 g gereinigtes Produkt mit einem Schmelzpunkt von 147 bis 155 °C erhalten, optischer Drehwert [α]²⁰_{D}:-26.2°.

### B) Herstellung der Titelverbindung

1 g des vorstehend erhaltenen Produktes wurden nach der in Beispiel 1E) beschriebenen Methode umgesetzt. Es wurden 0,47 g der Titelverbindung mit einem Schmelzpunkt von 201 bis 209 °C erhalten, optischer Drehwert [α]²⁰_{D}:-45.8°.

### Beispiel I:

| | |
|---|---|
| [(1'R),2R,3S,4S,5R,6R,9R,11R,12R,14R]-11-(1'-Hydroxypropyl)-3-[(2,6-dideoxy-3-C-methyl-3-C-methyl-α-L-ribohexopyranosyl)-oxy]-5-[(3,4,6-trideoxy-3(N-methyl-N-isopropylamino)-β-D-xylohexopyranosyl)-oxy]-2,4,6,8,11,14-hexamethyl-10,13,15-trioxatricyclo[9.2.1.1^{9.6}]-pentadecan-1-on (= Isomerengemisch der (Verbindung der Formel I, R¹ = Methyl) | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

## Patentansprüche

1. [(1'R),2R,3S,4S,5R,6R,9R,11R,12R,14R]-11-(1'-Hydroxypropyl) -2,4,6,8,11,14-hexamethyl-10,13,15-trioxatricyclo-[9.2.1.1^{9.6}]-pentadecan-1-on-Derivate der allgemeinen Formel I 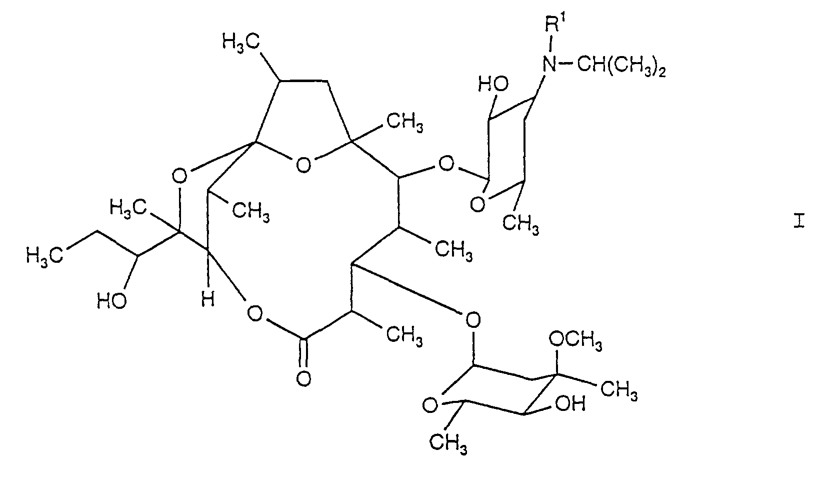 worin R¹ Methyl oder Wasserstoff bedeutet, und deren stabile und physiologisch verträgliche Säureadditionssalze.

2. Verbindungen gemäß Anspruch 1, worin R¹ Methyl bedeutet.

3. Arzneimittel, enthaltend eine pharmakologisch wirksame Menge einer Verbindung gemäß Anspruch 1 und übliche pharmazeutische Hilfs- und/oder Trägerstoffe.

4. Verfahren zur Herstellung von [(1'R),2R,3S,4S,5R,6R, 9R,11R,12R,14R]-11-(1'-Hydroxypropyl)-2,4,6,8,11,14-hexamethyl-10,13,15-trioxatricyclo[9.2.1.1^{9.6}] -pentadecan-1-on-Derivaten der allgemeinen Formel I, 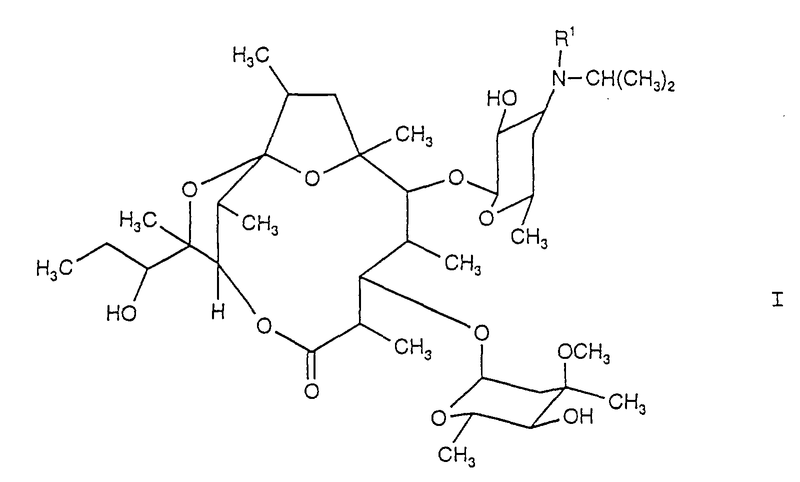 worin R¹ Methyl oder Wasserstoff bedeutet, und deren stabilen und physiologisch verträglichen Säureadditionssalzen, **dadurch gekennzeichnet, daß** man [2R(2'R,3'R),3S,4S,5R,6R,10R,11R]-11-(2',3'-Dihydroxypent-2'-yl)-2,4,6,8,10-pentamethyl-12,13-dioxabicyclo[8.2.1]tridec-8-en-1-on-Derivate der allgemeinen Formel II 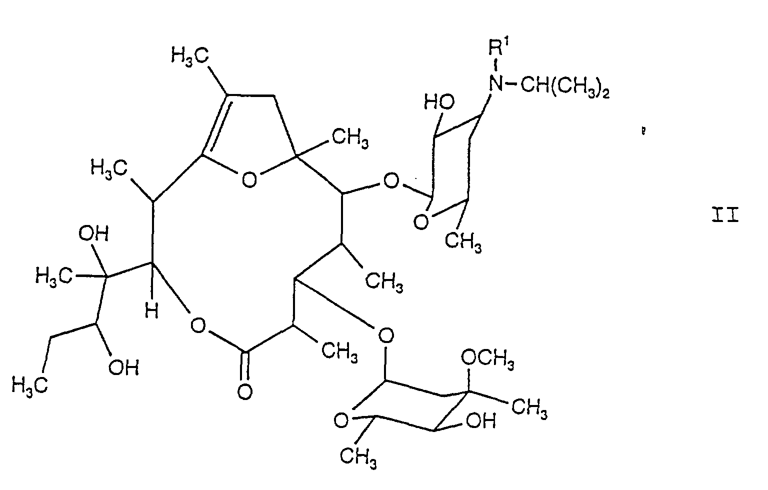 worin R¹ obige Bedeutung besitzt, durch Säurebehandlung in eine Verbindung der Formel I überführt und gewünschtenfalls in die erhaltene Verbindung der Formel I, worin R¹ Wasserstoff bedeutet, einen Methylrest R¹ einführt oder in der erhaltenen Verbindung der Formel I, worin R¹ Methyl bedeutet, den Methylrest R¹ abspaltet und gewünschtenfalls freie Verbindungen der Formel I in ihre stabilen Säureadditionssalze überführt oder die Säureadditionssalze in die freien Verbindungen der Formel I überführt.

## Claims

1. [(1'R),2R,3S,4S,5R,6R,9R,11R,12R,14R]-11-(1'-hydroxypropyl)-2,4,6,8,11,14-hexamethyl-10,13,15-trioxatricyclo[9.2.1.1^{9.6}]-pentadecan-1-one derivatives of the general formula I 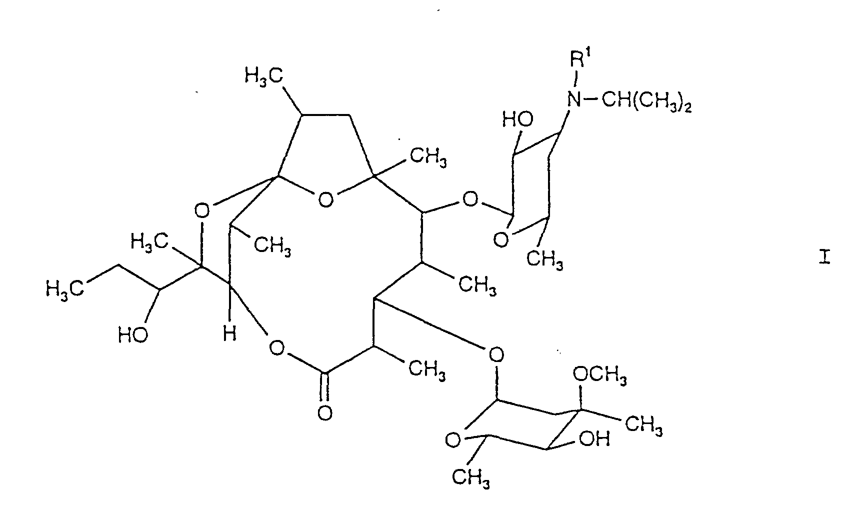 in which R¹ denotes methyl or hydrogen, and the stable and physiologically tolerated acid addition salts thereof.

2. Compounds according to Claim 1, wherein R¹ is methyl.

3. Medicament, containing a pharmacologically effective amount of a compound according to Claim 1 and conventional pharmaceutical auxiliary substances and/or vehicles.

4. Method for the preparation of [(1'R),2R,3S,4S,5R,6R,9R,11R,12R,14R]-11-(1'-hydroxypropyl)-2,4,6,8,11,14-hexamethyl-10,13,15-trioxatricyclo[9.2.1.1^{9.6}]-pentadecan-1-one derivatives of the general formula I 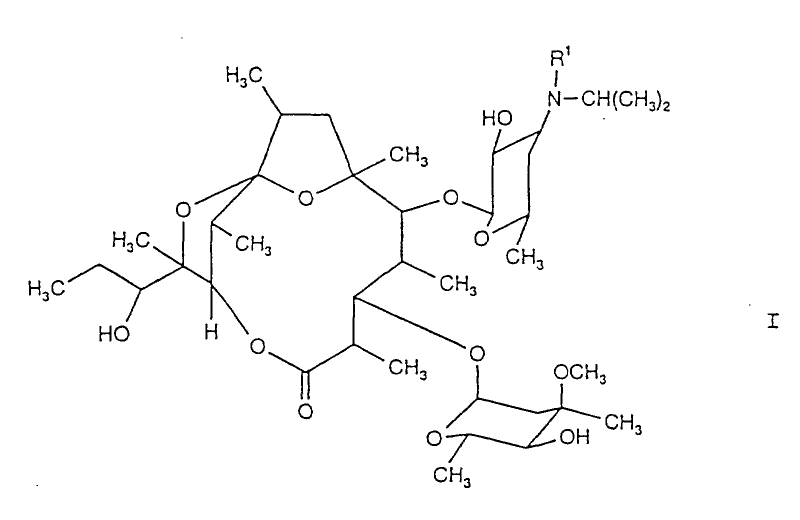 in which R¹ denotes methyl or hydrogen, and the stable and physiologically tolerated acid addition salts thereof, **characterised in that** [2R(2'R,3'R),3S,4S,5R,6R,10R,11R]-11-(2',3'-dihydroxypent-2'-yl)-2,4,6,8,10-pentamethyl-12,13-dioxabicyclo[8.2.1]-tridec-8-en-1-one derivatives of the general formula II 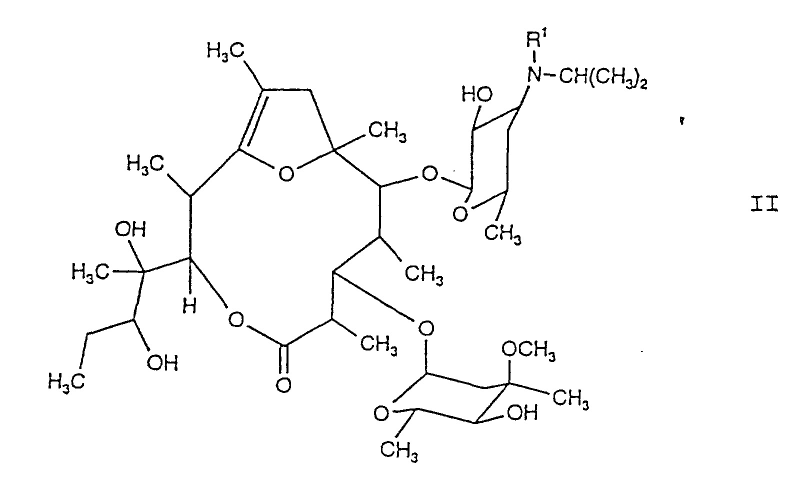 in which R¹ has the above meaning, are converted by acid treatment into a compound of Formula I and, if desired, a methyl radical R¹ is introduced into the resulting compound of Formula I in which R¹ denotes hydrogen, or the methyl radical R¹ is cleaved off in the resulting compound of Formula I in which R¹ denotes methyl, and, if desired, free compounds of Formula I are converted into their stable acid addition salts, or the acid addition salts are converted into the free compounds of Formula I.

## Revendications

1. Dérivés de la [(1'R),2R,3S,4S,5R,6R,9R,11R,12R,14R]-11-(1'-hydroxypropyl)-2,4,6,8,11,14-hexaméthyl-10,13,15-trioxatricyclo[9.2.1.1^{9.6}]-pentadécane-1-one, de formule générale I : 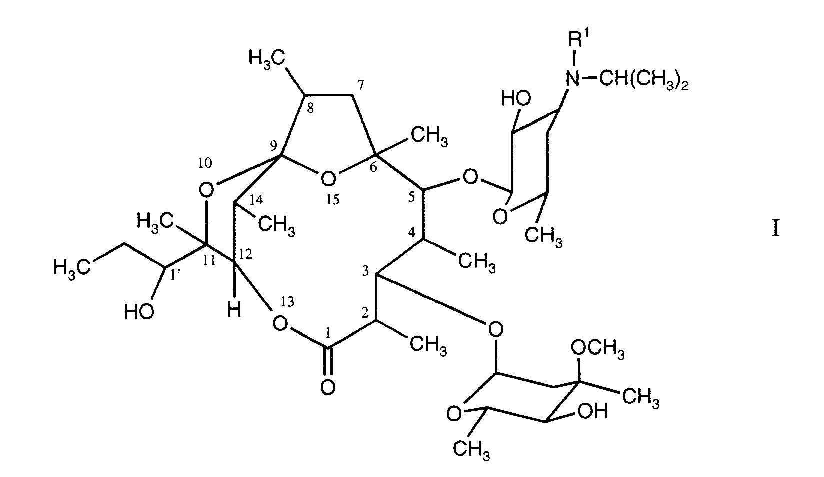 dans laquelle R¹ représente un groupe méthyle ou un atome d'hydrogène et leurs sels d'addition d'acides, stables et physiologiquement tolérables.

2. Composés selon la revendication 1, dans laquelle R¹ représente un groupe méthyle.

3. Médicament, contenant une quantité pharmacologiquement efficace d'un composé selon la revendication 1, et des adjuvants, des excipients, des supports et/ou véhicules pharmaceutiques usuels.

4. Procédé de préparation de dérivés de la [(1'R),2R,3S,4S,5R,6R,9R,11R,12R,14R]-11-(1'-hydroxypropyl)-2,4,6,8,11,14-hexaméthyl-10,13,15-trioxatricyclo[9.2.1.1^{9.6}]-pentadécane-1-one, de formule générale I 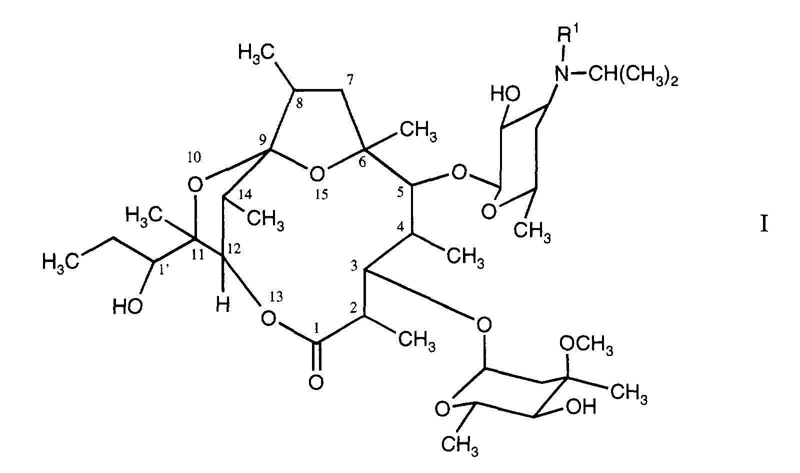 dans laquelle R¹ représente un groupe méthyle ou un atome d'hydrogène et de leurs sels d'addition d'acides stables et physiologiquement tolérables, procédé **caractérisé en ce qu'**on transforme, par traitement à l'aide d'un acide, des dérivés de [2R(2'R,3'R),3S,4S,5R,6R,10R,11R]-11-(2',3'-dihydroxypent-2'-yl)-2,4,6,8,10-pentaméthyl-12,13-dioxabicyclo[8.2.1]tridéc-8-ène-1-one, de formule générale II 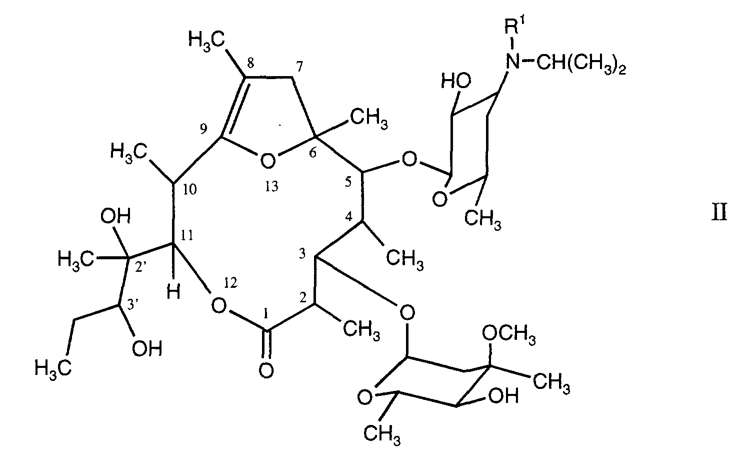 dans laquelle R¹ a le sens ci-dessus, en un composé de formule I et, si on le souhaite, on introduit dans le composé de formule I, dans laquelle R¹ représente un atome d'hydrogène, un reste méthyle R¹ ou bien, dans le composé obtenu de formule I, dans laquelle R¹ représente un groupe méthyle, on scinde le reste méthyle R¹ et, si on le souhaite, on transforme les composés libres de formule I en leurs sels d'addition d'acides stables ou bien on transforme les sels d'addition d'acides en composés libres de formule I.
